# EUROPEAN PATENT SPECIFICATION

(11) **EP 4 522 043 B1**
(45) Date of publication and mention of the grant of the patent: **25.11.2026**
(21) Application number: 23723211.1
(22) Date of filing: 11.05.2023
(51) Int. Cl.: A61B 17/22, A61M 25/00

(54) **FUNNEL CATHETER TIP WITH ANGLED FOLDING HOOPS**
TRICHTERKATHETERSPITZE MIT ABGEWINKELTEN KLAPPBAREN SCHLAUFEN
POINTE DE CATHÉTER EN ENTONNOIR AVEC ARCEAUX PLIANTS INCLINÉS

(30) Priority: 12.05.2022 US 202263341145 P; 25.04.2023 US 202318138801
(43) Date of publication of application: 19.03.2025
(73) Proprietor: Neuravi Limited, Galway H91 K5YD (IE)
(72) Inventor: KEATING, Karl, Galway, H91 K5YD (IE); VALE, David, Galway, H91 K5YD (IE); KELLY, Ronald, Galway, H91 K5YD (IE)
(74) Representative: Carpmaels & Ransford LLP
(86) International application number: PCT/EP2023/062609
(87) International publication number: WO 2023/217966

(56) References cited:
- WO-A1-2012/156924
- WO-A1-2015/189354
- US-A1- 2005 038 447
- US-A1- 2010 036 312
- US-A1- 2021 153 884
- US-A1- 2021 275 197
- US-A1- 2022 125 450

## Description

### Field of Invention

The present invention generally relates to devices for removing acute blockages from blood vessels during intravascular medical treatments. More specifically, the present invention relates to retrieval catheters with expandable tips into which an object or objects can be retrieved.

### Background

Clot retrieval aspiration cathetersfne and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and highly tortuous. Traditional devices are often either too large in profile, lack the deliverability and flexibility needed to navigate particularly tortuous vessels, or are ineffective at removing a clot when delivered to the target site.

Many existing designs for aspiration retrieval catheters are often restricted to, for example, inner diameters of 6Fr or between approximately 0.068 to 0.074 inches. Larger sizes require an even larger guide or sheath to be used, which then necessitates a larger femoral access hole to close. Most physicians would prefer to use an 8F guide / 6F sheath combination, and few would be comfortable going beyond a 9F guide / 7F sheath combination (1 French = 1/3 mm; 1 inch ≈ 2.54 cm). This means that once at the target site, a clot can often be larger in size than the inner diameter of the aspiration catheter and must otherwise be compressed to enter the catheter mouth. This compression can lead to bunching up and subsequent shearing of the clot during retrieval. Firm, fibrin-rich clots can also become lodged in the fixed-mouth tip of these catheters making them more difficult to extract. This lodging can result in softer portions breaking away from firmer regions of the clot.

Small diameters and fixed tip sizes are also less efficient at directing the aspiration necessary to remove blood and thrombus material during the procedure. The suction must be strong enough such that any fragmentation that may occur as a result of aspiration or the use of a mechanical thrombectomy device cannot migrate and occlude distal vessels. When aspirating with a fixed-mouth catheter, a significant portion of the aspiration flow ends up coming from vessel fluid proximal to the tip of the catheter, where there is no clot. This significantly reduces aspiration efficiency, lowering the success rate of clot removal.

Intermediate- and aspiration-catheters and/or those with expandable tips are therefore desirable because they provide a large lumen and distal mouth to accept a clot with minimal resistance. However, current designs with fixed braids, frames, or expanded sizes do not allow for any additional expansion of the tip which can complicate the interaction with and reception of a clot during ingestion.

Document WO 2012/156924 Al describes an apparatus for aspirating blood from a blood vessel through a catheter located in the blood vessel, the apparatus comprising: a first tube portion having a configuration for which the first portion has an outer diameter substantially equal to an inner diameter of the catheter; and a second tube portion connected to the first tube portion, which second tube portion is expandable to occlude the blood vessel.

Document US 2021/0275197 A1 describes a system that has an outer catheter and an inner aspirating clot retrieval catheter having an expansile distal tip for flow restriction, and a large mouth into which a clot or other obstructions can be retrieved. The clot retrieval catheter can have a support tube proximal of the tip. The expansile tip can be a strut framework, and a flexible, low-modulus cover is disposed around at least a portion of the tip strut framework and the proximal support tube. The distal end of the tip can be encapsulated by a low-friction elastomeric lip for atraumatic contact with the walls of a blood vessel. The tip has a collapsed delivery configuration and expands radially into a deployed configuration.

Document US 2022/0125450 Al describes an aspiration catheter for removing clot from a blood vessel includes a catheter body having a scaffold extending distally from a distal end of the body. An aspiration lumen runs from the distal end to a proximal end of the body, and a central clot-receiving passage in the scaffold is contiguous with the aspiration lumen of the catheter body. A vacuum-resistant membrane covers the scaffold and establishes a clot aspiration path from a distal end of the scaffold to a proximal end of the aspiration lumen, in the catheter body so that applying a vacuum to the proximal end of the aspiration lumen can draw clot into the central clot-receiving passage. The scaffold may have a comical configuration, a cylindrical configuration, or a combination thereof, and at least a distal portion of the scaffold is radially expandable from a delivery configuration to an extraction configuration.

Document WO 2015/189354 A1 describes a catheter that provides a proximal seal against a guide catheter inner lumen so that aspiration may be applied through a guide catheter and thus take advantage of the large proximal lumen. The catheter is a rapid exchange (Rx) catheter with an exit port that defines a transfer port for aspiration. Aspiration can be applied to the lumen of the guide catheter and is directed to and effective at the tip of the Rx aspiration catheter due to the presence of flow a restrictor or seal between the outer surfaceof the Rx aspiration catheter and the inner guide catheter. This seals the lumen between the outside of the Rx aspiration catheter and the inner surface of the guide catheter and prevents backflow of blood into the tip of guide catheter reducing the effectiveness of the aspiration. An expansile tip of the Rx catheter facilitates aspiration and retrieval of the clot by expanding under load. The expansile tip can also partially or fully occlude the vessel providing flow arrest and thereby improving aspiration effectiveness.

Document US 2005/0038447 Al describes a device for removing blood clots and methods of making and using the same. The clot pulling device may include a first spine, a second spine disposed parallel to the first spine, and a basket disposed between and coupled to the spines. In addition, a pushing member may be coupled to the first spine and extend proximally therefrom.

Document US 2021/0153884 Al describes a clot retrieval catheter with an expansile distal tip and a large, distal facing mouth into which clots or other obstructions can be retrieved. The tip can be formed from a distal ring of leaflets or hoops. One or more pull cables can be retracted to actuate and radially expand the expansile distal tip. The clot retrieval catheter can have a catheter shaft with flexibility-adding features proximal of the tip. The catheter shaft can be a multi-lumen configuration with a large catheter lumen for passing auxiliary devices and directing aspiration and one or more guide lumens which can route the pull cables to the leaflets or hoops of the expansile tip. A flexible, low-modulus membrane can be disposed around at least a portion of the expansile tip and catheter shaft.

Document US 2010/0036312 Al describes an apparatus for removing material within a body lumen that includes a catheter including a proximal end, a distal end for introduction into a body lumen, and an aspiration lumen extending therebetween; a guide member extending from the distal end and terminating in a distal tip, the guide member comprising a track adjacent a track lumen extending from the distal tip into the aspiration lumen; and an obstruction clearing device deployable from the guide member and retractable along the track. In addition or alternatively, the apparatus includes a cutting head reciprocable within the aspiration lumen adjacent the distal end for macerating material being aspirated into the aspiration lumen.

### Summary

It is an object of the present designs to provide devices to meet the above-stated needs. The designs can be for a clot retrieval catheter capable of remove a clot from cerebral arteries in patients suffering AIS, from coronary native or graft vessels in patients suffering from MI, and from pulmonary arteries in patients suffering from PE and from other peripheral arterial and venous vessels in which a clot is causing an occlusion.

The invention is defined by claim 1. Further embodiments of the invention are defined by the dependent claims.

One example of the present disclosure provides a catheter. The catheter can include an elongate body having a longitudinal axis, an inner diameter, and a distal end. The catheter can include a support frame connected at the distal end of the elongate body. The support frame can have a collapsed delivery configuration, an expanded deployed configuration, and a framework of struts including a plurality of distal hoop segments. At least a portion of the distal hoop segments can fold distally in the collapsed delivery configuration such that a collapsed inner diameter of the support frame is approximately equal to the inner diameter of the elongate body. The support frame can have a maximum outer diameter in the expanded deployed configuration. The maximum outer diameter can be less than an inner diameter of a target vessel at a treatment site.

A first more distal hoop segment of the plurality of distal hoop segments can have a first diameter when the support frame is in the expanded deployed configuration, and a second more proximal hoop segment of the plurality of distal hoop segments can form a second diameter when the support frame is in the expanded deployed configuration. The first diameter can be greater than the second diameter.

The struts of the distal hoop segments can have a curvilinear profile in the collapsed delivery configuration.

The support frame can have a longitudinal length sized to be less than three times the inner diameter of the elongate body.

The catheter can include one or more connecting spines connecting the distal hoop segments of the support frame with the elongate body.

The plurality of distal hoop segments can fold so the support frame has a collapsed inner diameter in the collapsed delivery configuration approximately equal to the inner diameter of the elongate body.

The plurality of distal hoop segments can include distally unconnected peaks which move distally when the support frame is folded to the collapsed delivery configuration.

The distally unconnected peaks can move proximally when the support frame is in the expanded deployed configuration.

A first more distal hoop segment of the plurality of distal hoop segments can form a first folded angle with respect to the longitudinal axis when the support frame is in the collapsed delivery configuration. A second more proximal hoop segment of the plurality of distal hoop segments can form a second folded angle with respect to the longitudinal axis when the support frame is in the collapsed delivery configuration. The first folded angle can be less than the second folded angle.

The plurality of distal hoop segments can form a series of rings concentric with the longitudinal axis when the support frame is in the expanded deployed configuration.

At least a portion of each of the plurality of distal hoop segments can form an acute angle with respect to the longitudinal axis when the support frame is in the collapsed delivery configuration.

Each hoop segment of the plurality of distal hoop segments can have a non-planar cross section when the support frame is in the collapsed delivery configuration.

One or more hoops of the plurality of distal hoop segments can include one or more axial curves when the support frame is in the collapsed delivery configuration.

The support frame can include a plurality of ribs and one or more axial spines. At least one of the one or more axial spines can be aligned with a connecting spine of the support frame.

Another example of the present disclosure provides a catheter. The catheter can have a longitudinal axis. The catheter can include a support frame having a collapsed delivery configuration, an expanded deployed configuration, and a framework of struts including one or more pairs of opposing C-shaped petals. Each pair of opposing C-shaped petals can include a proximal petal and a distal petal. The proximal petal and the distal petal can be folded to an axially-elongated profile in the collapsed delivery configuration. The support frame can have a maximum outer diameter in the expanded deployed configuration. The maximum outer diameter can be less than an inner diameter of a target vessel at a treatment site.

The opposing C-shaped petals can be located (clocked) at 90 degrees to one another.

The opposing C-shaped petals can include distally unconnected peaks.

At least one of the distally unconnected peaks can include a circumferential undulation.

At least one of the opposing C-shaped petals can include an intermediate hoop.

The support frame further can include a proximal ring member connected to a distal end of an elongate body of the catheter.

The plurality of distal hoop segments can have a planar cross section in the expanded deployed configuration.

The support frame can have a collapsed length in the collapsed delivery configuration and an expanded length in the expanded deployed configuration. The collapsed length can be greater than the expanded length.

At least one hoop segment of the plurality of distal hoop segments can define a plane passing through at least a portion of a perimeter of the hoop segment. The plane can form an acute angle with respect to the longitudinal axis when the distal end is in the collapsed delivery configuration.

The distal end of the support frame in the expanded deployed configuration can have a circular profile including a center radially offset from the longitudinal axis of the elongate body.

In some examples, the hoop segments themselves can form cells of the support frame and can bend proximally or distally to fold to a smaller diameter in the collapsed delivery configuration. In one example, the hoop segments can be one or more pairs of opposing C-shaped petals which can be heat set or sprung to expand radially outward when the expandable tip is deployed. In another example, the hoop segments form two longitudinally-opposed rows connected by one or more axial spines. In a further example, the hoop segments can be in the shape of broad loops joined by a single axial spine.

In these examples, when the frame is expanded and the hoops flex radially outward, a first radial size of a more distal hoop or pair of opposing hoops can be greater than a second radial size of an adjacent, more proximal hoop or set of opposing hoops. This can allow the expanded frame to form a substantially funnel shape as the radial size is incrementally increased in more distal hoops. In other examples, at least one of the one or more hoop segments can have a distal peak that does not share a connection with another hoop segment, allowing for independent flexing and a greater range of motion.

Other aspects of the present disclosure will become apparent upon reviewing the following detailed description in conjunction with the accompanying figures. Additional features or manufacturing and use steps can be included as would be appreciated and understood by a person of ordinary skill in the art.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation. It is expected that those of skill in the art can conceive of and combine elements from multiple figures to better suit the needs of the user.
FIG. 1 is a diagram of a clot retrieval catheter with an expandable support frame being advanced through the vasculature according to aspects of the present invention;
FIG. 2A is a perspective view of a clot retrieval catheter with an expandable support frame in a collapsed delivery configuration, according to aspects of the present invention;
FIG. 2B is a side view of the clot retrieval catheter of FIG. 2A with the expandable support frame in the collapsed delivery configuration, according to aspects of the present invention;
FIG. 2C is a top view of the clot retrieval catheter of FIGs. 2A and 2B with the expandable support frame in the collapsed delivery configuration, according to aspects of the present invention;
FIG. 2D is a side view of the clot retrieval catheter of FIGs. 2A-2C with the expandable support frame in an expanded deployed configuration, according to aspects of the present invention;
FIG. 3A is a top view of another clot retrieval catheter with an expandable support frame in a collapsed delivery configuration, wherein the shaft of the catheter includes slanted ribs, according to aspects of the present invention;
FIG. 3B is a side view of the clot retrieval catheter of FIG. 3A with the expandable support frame in the collapsed delivery configuration, according to aspects of the present invention;
FIG. 3C is a top view of the clot retrieval catheter of FIGs. 3A and 3B with the expandable support frame in an expanded deployed configuration, according to aspects of the present invention;
FIG. 4A is a perspective view of a clot retrieval catheter with an expandable support frame in a collapsed delivery configuration, wherein the shaft of the catheter includes an interrupted spine, according to aspects of the present invention;
FIG. 4B is a side view of the clot retrieval catheter of FIG. 4A with the expandable support frame in the collapsed delivery configuration, according to aspects of the present invention;
FIG. 4C is a top view of the clot retrieval catheter of FIGs. 4A and 4B with the expandable support frame in the collapsed delivery configuration, according to aspects of the present invention;
FIG. 4D is a top view of the clot retrieval catheter of FIGs. 4A-4C with the expandable support frame in an expanded deployed configuration, according to aspects of the present invention;
FIG. 5A is a top view of a clot retrieval catheter with an expandable support frame in an expanded deployed configuration, wherein the catheter includes a base ring, according to aspects;
FIG. 5B is a top view of the clot retrieval catheter of FIG. 5A with the expandable support frame in a collapsed delivery configuration, according to aspects;
FIG. 5C is a side view of the clot retrieval catheter of FIGs. 5A and 5B with the expandable support frame in the collapsed delivery configuration, according to aspects;
FIG. 5D is a perspective view of the clot retrieval catheter of FIGs. 5A-5C with the expandable support frame in the collapsed delivery configuration, according to aspects;
FIG. 6A is a side perspective view of a clot retrieval catheter with an expandable support frame in a collapsed delivery configuration, wherein the shaft of the catheter includes spine connectors, according to aspects of the present invention;
FIG. 6B is a side view of the clot retrieval catheter of FIG. 6A with the expandable support frame in the collapsed delivery configuration, according to aspects of the present invention;
FIG. 6C is a top view of the clot retrieval catheter of FIGs. 6A and 6B with the expandable support frame in the collapsed delivery configuration, according to aspects of the present invention;
FIG. 6D is a side perspective view of the clot retrieval catheter of FIGs. 6A-6C with the expandable support frame in an expanded deployed configuration, according to aspects of the present invention;
FIG. 7A is a side perspective view of a clot retrieval catheter with an expandable support frame in a collapsed delivery configuration, wherein the shaft of the catheter includes spine connectors, according to aspects of the present invention;
FIG. 7B is a side view of the clot retrieval catheter of FIG. 7A with the expandable support frame in the collapsed delivery configuration, according to aspects of the present invention;
FIG. 7C is a top view of the clot retrieval catheter of FIGs. 7A and 7B with the expandable support frame in the collapsed delivery configuration, according to aspects of the present invention;
FIG. 7D is a side perspective view of the clot retrieval catheter of FIGs. 7A-7C with the expandable support frame in an expanded deployed configuration, according to aspects of the present invention;
FIG. 8A is a perspective view of a clot retrieval catheter with an expandable support frame in a collapsed delivery configuration, wherein the support frame includes C-shaped petals, according to aspects of the present invention;
FIG. 8B is a perspective view of the clot retrieval catheter of FIG. 8A with the expandable support frame in an expanded deployed configuration, according to aspects of the present invention;
FIG. 9 is a side perspective view of a clot retrieval catheter with an expandable support frame in a collapsed delivery configuration, wherein the support frame includes a circumferential undulation, according to aspects of the present invention; and
FIG. 10 is a perspective view of a clot retrieval catheter with an expandable support frame and a jacket in an expanded deployed configuration, according to aspects of the present invention.

### Detailed Description

Specific examples of the present invention are now described in detail with reference to the Figures, where identical reference numbers indicate elements which are functionally similar or identical. The examples address many of the deficiencies associated with traditional clot retrieval aspiration catheters, such as poor or inaccurate deployment to a target site and ineffective clot removal.

The designs herein can be for a clot retrieval catheter with a lumen and a distal tip that can expand to a diameter larger than that of the guide or sheath through which it is delivered when advanced beyond the distal end. The designs can have a proximal elongate body for the shaft of the catheter, and a distal tip with an expanding support frame and, in some examples, a jacket (e.g., a polymeric jacket) to give the tip atraumatic properties and the ability to flexibly expand further in the expanded deployed configuration when ingesting a clot. The catheter frame and tip can be sufficiently flexible to navigate highly tortuous areas of the anatomy and be able to recover and maintain the inner diameter of the lumen when displaced in a vessel.

Accessing the various vessels within the vascular, whether they are coronary, pulmonary, or cerebral, involves well-known procedural steps and the use of a number of conventional, commercially-available accessory products. These products, such as angiographic materials, mechanical thrombectomy devices, microcatheters, and guidewires are widely used in laboratory and medical procedures. When these products are employed in conjunction with the devices in the description below, their function and exact constitution are not described in detail. While the description is in many cases in the context of thrombectomy treatments in intercranial arteries, the disclosure may be adapted for other procedures and in other body passageways as well.

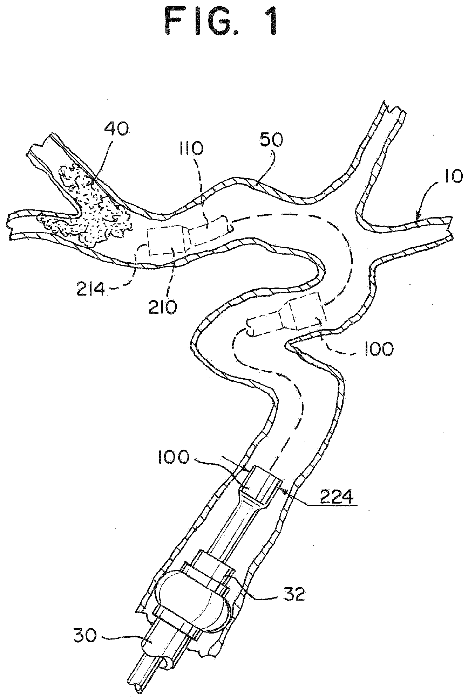

Turning to the figures, FIG. 1 illustrates a possible sequence for approaching an occlusive clot 40 using a clot retrieval catheter 100 of the designs disclosed herein. The clot 40 can be approached with the catheter 100 collapsed within a guide sheath 30 or other access catheter. When the vasculature 10 becomes too narrow and/or tortuous for further distal navigation with the guide sheath 30, the catheter 100 can be deployed for further independent travel distally. The catheter 100 can be highly flexible such that it is capable of navigating the M1 or other tortuous regions of the neurovascular to reach an occlusive clot, and can have an expanded outer diameter slightly less than that of the target vessel so the catheter is capable of distal navigation independently after deployment.

The clot retrieval catheter 100 can have a flexible elongate body 110 serving as a shaft with a large internal bore (which in some cases can be 0.080 inches or larger) and a distal tip section having a collapsible support frame 210. They can help the catheter to be delivered to a target site by a variety of methods, including over a guidewire, over a microcatheter, with a dilator/access tool, or by itself. 1 inch ≈ 2.54 cm. 1 French = 1/3 mm.

In many cases, the design of the tip of the support frame 210 can be configured so that the entire catheter 100 can be delivered through (and retrieved back through) common standard 6F sheaths/8F guides, which typically have inner lumens of less than 0.090 inches. The tip can self-expand once advanced to an unconstrained position distal to the distal end 32 of the guide sheath 30. As the catheter can be deployed proximal to, and can then be advanced independently to, a remote occlusion, the support frame 210 of the tip is designed to resist collapse from the forces of aspiration, have excellent lateral flexibility in both the expanded and collapsed states, and an atraumatic profile to prevent snagging on bifurcations in vessels.

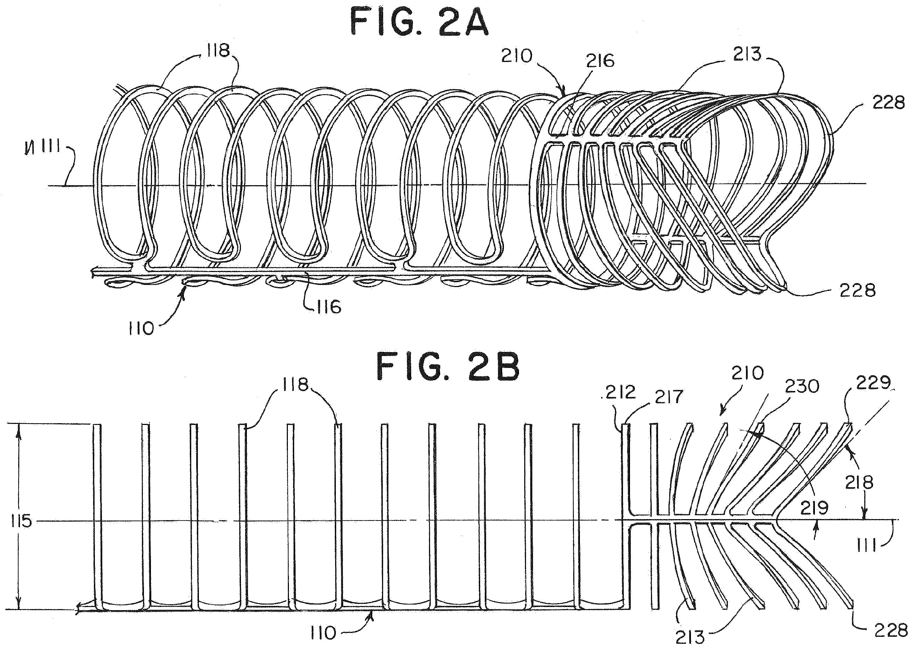

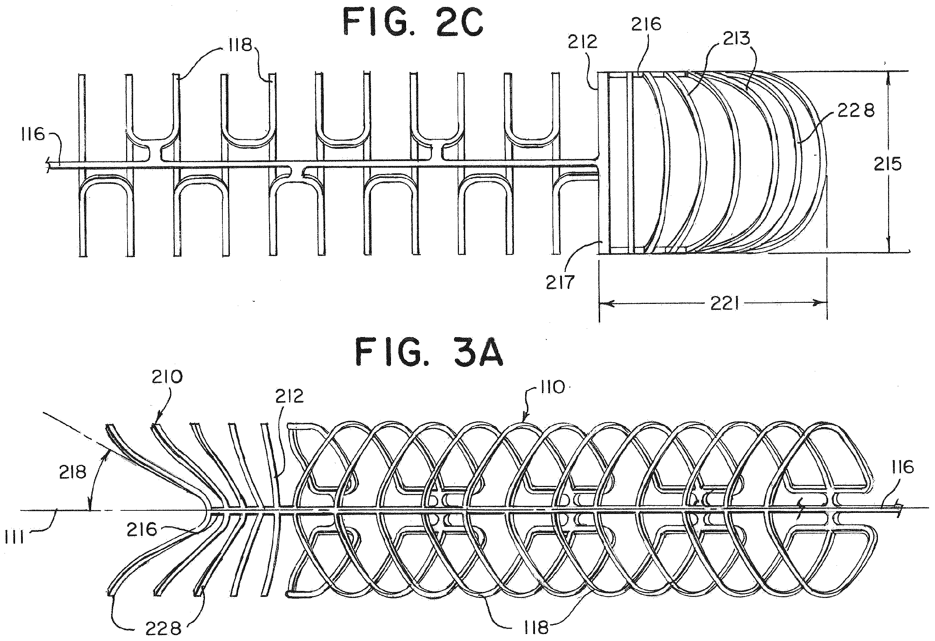

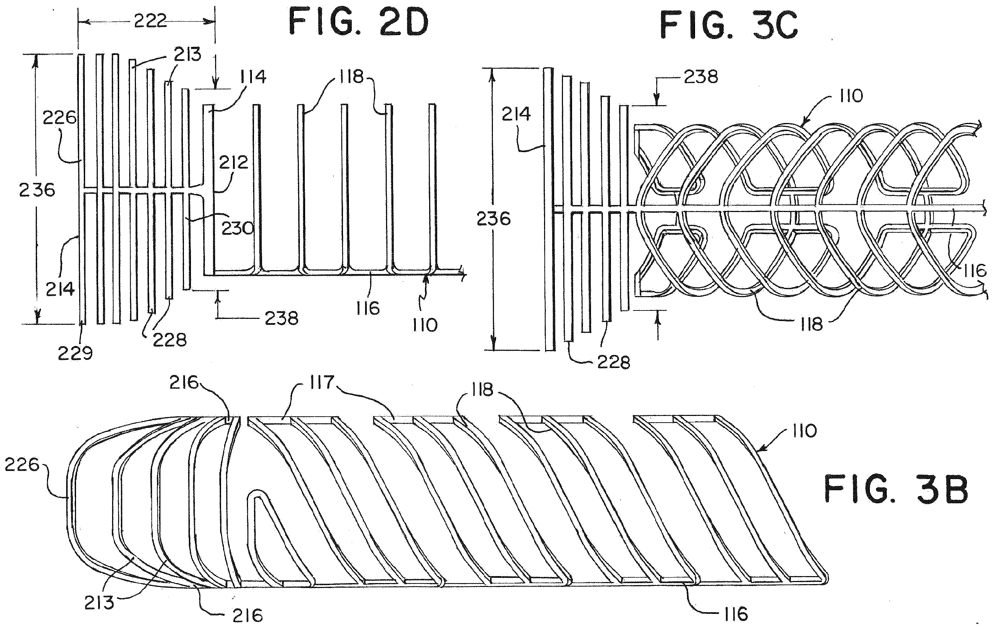

FIGs. 2A through 2D provide views of an example clot retrieval catheter 100 with an expandable support frame 210 in both collapsed delivery configurations (see FIGs. 2A through 2C) and an expanded deployed configuration (see FIG. 2D). The catheter 100 can include an elongate body 110 at a proximal end (i.e., to the left on FIG. 2A) that has a longitudinal axis 111, an inner diameter 115, and a distal end. The catheter 100 can include the support frame 210 connected at the distal end of the elongate body 110, a proximal end 212 of the support frame 210 being proximate the distal end of the elongate body 110. The support frame 210 can have a collapsed delivery configuration, an expanded deployed configuration, and a framework of struts comprising a plurality of distal hoop segments 213. A distal end of the support frame 210 can open in the expanded, deployed configuration at the target site in a vessel. The proximal end 212 of the support frame 210 can define a proximal ring 217 that can provide support to the support frame 210 at the junction of the support frame 210 and the elongate body 110.

The structure for the elongate body 110 can define the catheter lumen, which can be used for the delivery of auxiliary devices, contrast injection, and direct distal aspiration to a clot face through the support frame 210. The underlying structure of the elongate body 110 can be, for example, a frame cut from a hypotube of a superelastic material with shape memory properties, such as Nitinol with an internal low friction liner and outer polymer jacket or jackets 180 that can be reflowed into the structure during manufacturing. Alternately, a more traditional polymer and/or metal braid or coil support structure can be used, or some combination of these.

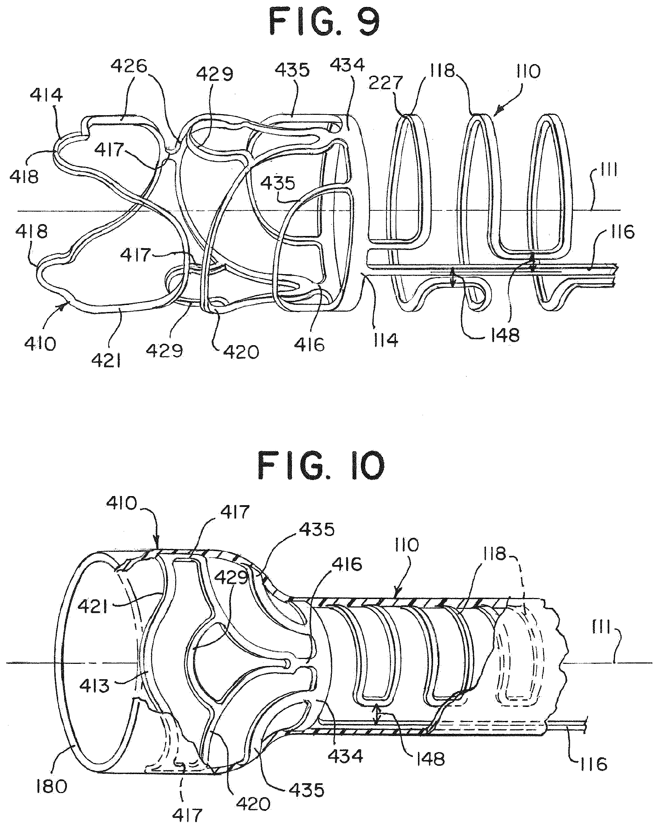

The outer surface of the elongate body 110 and support frame 210 can be at least partially covered by a jacket or jackets 180. FIG. 10 provides an example view of the jacket 180, but it will be appreciated that any example catheter 100 described herein can include a jacket 180. The jacket 180 can block proximal fluid from entering the expanded tip during aspiration and retrieval of the clot, allowing for more efficient direction of the aspiration force while preventing the distal migration of clot fragments or other debris during the procedure. In one example, the jacket 180 can be formed from a highly-elastic material such that the radial force exerted by expanding the expansile tip is sufficient to stretch the membrane to the funnel shape contours of the tip when in the expanded deployed configuration. One example can be using a ductile elastomer which has the advantages of being soft and flexible with resistance to tearing and perforation due to a high failure strain. Alternately, the jacket 180 can be loosely fitted to the catheter 100 and fold over the support frame 210 edges so that the support frame 210 can move more freely when expanded and collapsed.

As mentioned, the elongate body 110 can be sized to be compatible with relatively low-profile guide sheaths 30 and catheters, so that a puncture wound in the patient's groin (in the case of femoral access) can be easily and reliably closed. For example, the clot retrieval catheter 100 may be required to pass through the lumen of a guide sheath 30 with an inner diameter of less than 0.110 inches, for example 0.090 inches, or in some cases less than 0.087 inches, and preferably less than 0.085 inches. In other examples with a 5Fr sheath, the inner diameter can be less than 0.080 inches and can be as small as 0.070 inches. Therefore, the catheter 100 can have an overall delivery profile with an inner diameter 115 of approximately 0.067-0.074 inches (0.084 inch or 2 mm outer diameter), and yet be able to expand its distal tip and mouth at the support frame 210 to a size just smaller than the diameter of the vessel approximate where the clot is located, which can be as large as 5 mm. It will be appreciated that larger or smaller catheter shafts can be used if paired with a larger or smaller guide sheath 30. 1 inch ≈ 2.54 cm. 1 French = 1/3 mm.

The support frame 210 can similarly be sized for compatibility. The support frame 210 can have an inner diameter in the collapsed delivery configuration sized so that radial forces are not excessively high for delivery through the selected guide sheath 30, which can for example have an inner diameter of 0.085 inches. Once deployed beyond the distal end 32 of the guide sheath 30, the support frame 210 can grow radially such that the tip is just smaller in diameter than the target vessel. For example, a large size of approximately 3-5 mm for clots in the ICA, an intermediate size for proximal / large M1 clots of approximately 0.090-0.110 inches in diameter, and/or a small size for small/distal M1 clots of approximately 0.080-0.090 inches in diameter. In many situations, the tip can have a maximum inner diameter 115 within a range of approximately 0.080-0.120 inches in the expanded deployed configuration. The support frame 210 further comprising a longitudinal length 211 sized to be less than three times the inner diameter 115 of the elongate body 110. The support frame 210 can include a collapsed length 221 in the collapsed delivery configuration and an expanded length 222 in the expanded deployed configuration, the collapsed length being greater than the expanded length.

Referring again to FIGs. 2A through 2D, at least a portion of the distal hoop segments 213 can fold distally in the collapsed delivery configuration such that a collapsed inner diameter 215 of the support frame is approximately equal to the inner diameter 115 of the elongate body 110. The struts of the distal hoop segments 213 can have a curvilinear profile in the collapsed delivery configuration. The support frame 210 can have a maximum outer diameter 224 (e.g., diameter 236) in the expanded deployed configuration (see FIG. 2D) less than an inner diameter of a target vessel at a treatment site. In some examples, however, the support frame 210 can have a maximum outer diameter 224 (e.g., diameter 236) in the expanded deployed configuration that is slightly larger than the target vessel. In such a situation, the compliant distal support frame 210 can resize itself to the vessel, allowing it to be advanced to the clot. The elongate body 110 can have an axial spine 116 that extends along the length of the elongate body 110 parallel to the longitudinal axis 111. The spine 116 can provide structural support and define planes or axes about which the elongate body 110 can bend. The spine 116 can include one or more ribs 118 extending from the spine 116. The ribs 118 can curve to form a cylindrical shape such that the elongate body 110 is shaped as a lumen to aspirate and capture/remove a clot from the target site.

The distal hoop segments 213 can fold distally in the collapsed configuration so that the distal hoop segments 213 are angled toward the distal end of the catheter 100. These folds in the support frame 210 can unfold when the catheter 100 is expended into the expanded configuration. In some examples, the plurality of distal hoop segments 213 can have distally unconnected peaks 228 which move distally when the support frame 210 is folded to the collapsed delivery configuration, as shown in FIG. 2B. A first more distal hoop segment 229 of the plurality of distal hoop segments can have a first folded angle 218 with respect to the longitudinal axis 111 when the support frame 210 is in the collapsed delivery configuration; a second more proximal hoop segment 230 of the plurality of distal hoop segments 213 can have a second folded angle 219 with respect to the longitudinal axis 111 when the support frame 210 is in the collapsed delivery configuration. The first folded angle 218 can be less (e.g., smaller) than the second folded angle 219. In some examples, one or more of the distalmost hoop segments 213 (e.g., the distalmost hoop segment 226) can have an acute angle (e.g., angle 218) with respect to the longitudinal axis 111 when the support frame is in the collapsed delivery configuration. This configuration can enable the distal hoop segments 213 to unfold into the full-shaped, expanded configuration shown in FIG. 2D. The plurality of distal hoop segments 213 can form a series of rings concentric with the longitudinal axis 111 when the support frame 210 is in the expanded deployed configuration.

In certain examples, the support frame 210 can include one or more connecting spine(s) 216 from which the distal hoop segments 213 extend. In some examples, the connecting spine(s) 216 can be aligned with the axial spine 116 of the elongate body 110; in some examples, the connecting spine(s) 216 can be radially offset from the axial spine 116, as shown in FIG. 2A. A first more distal hoop segment 229 of the plurality of distal hoop segments 213 can have a first diameter 236 when the support frame 210 is in the expanded deployed configuration, and a second more proximal hoop segment 230 of the plurality of distal hoop segments 213 can have a second diameter 238 when the support frame 210 is in the expanded deployed configuration. The first diameter 236 can be a greater than the second diameter 238 to create the funnel shape for the support frame 210, as shown in FIG. 2D. The plurality of distal hoop segments 213 can have a planar cross section in the expanded deployed configuration.

FIGs. 3A through 3C provide views of an example clot retrieval catheter 100 with an expandable support frame 210 in both collapsed delivery configurations (see FIGs. 3A and 3B) and an expanded deployed configuration (see FIG. 3C). The example catheter 100 in FIGs. 3A through 3C is similar to the example shown for FIGs. 2A through 2D, but the example in FIGs. 3A through 3C provides a variation of the elongate body 110. As shown, the elongate body 110 can include an interrupted spine 117 positioned along the length (e.g., along the longitudinal axis 111) of the elongate body 110. The interrupted spine 117 can be positioned diametrically opposed the axial spine 116, and can join two or more ribs 118 together to create a more robust elongate body 110. To illustrate using the example shown in FIG. 3B the interrupted spine 117 includes three ribs 118 connected to each other, which are separated from the next set of three ribs 118 by the interruption of the interrupted spine 117.

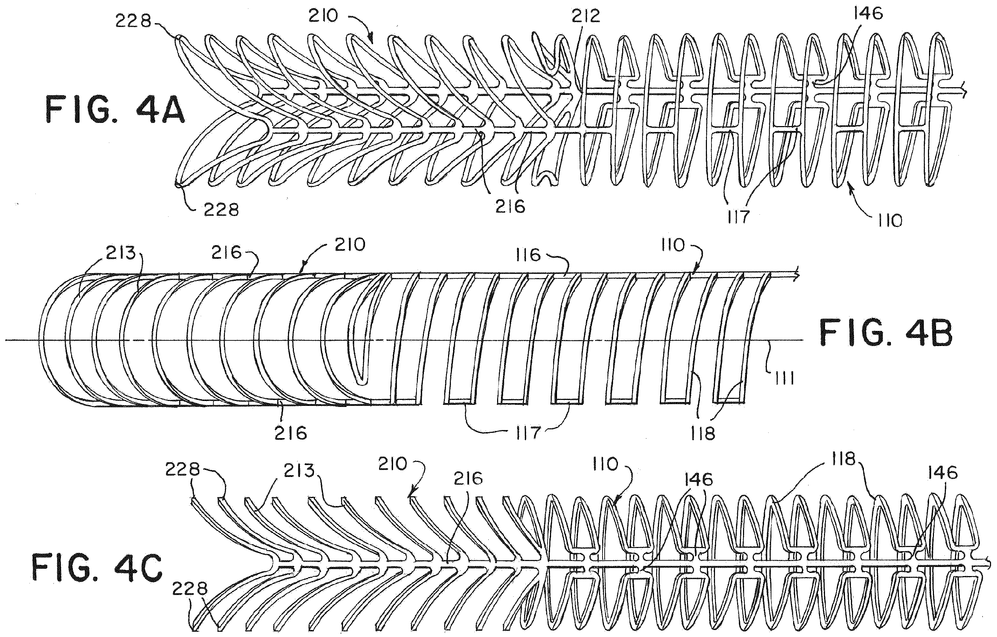

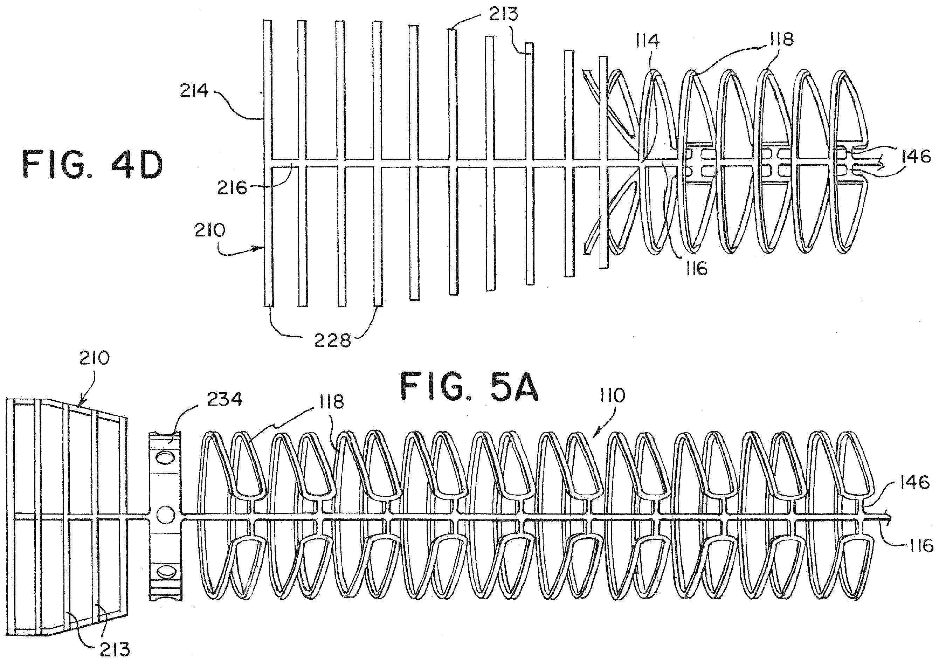

FIGs. 4A through 4D provide views of an example clot retrieval catheter 100 with an expandable support frame 210 in both collapsed delivery configurations (see FIGs. 4A through 4C) and an expanded deployed configuration (see FIG. 4D). The example catheter 100 in FIGs. 4A through 4D is similar to the example shown for FIGs. 3A through 3C, but the example in FIGs. 4A through 4D provides a variation of the elongate body 110. FIGs. 4A through 4D illustrate an example where the elongate body 110 framework has pairs of supporting ribs 118 which merge into a single spine connector 146 for connections with the axial spine 116. Each set of support ribs 118 can have one, two, three, or more ribs 118. By connecting support ribs 118 in sets that have a single connection to the one or more spines 116, additional flexibility can be gained by having a longer length of spine 116 which is free to bend for a given density of ribs 118. This arrangement can also reduce strain at the connection between the spine connectors 146 and the axial spine 116.

Alternately, a similar design can see a series of supporting ribs 118 merging into diametrically opposed spine connectors for connections with twin spines 116 spaced 180 degrees apart. Additional spines can also be envisioned which trade some lateral flexibility for better pushability than can be achieved with fewer spines. The additional axial stiffness can also help prevent the elongate body from stretching under tension, such as when an expandable tip is being drawn proximally into an outer sheath with a firm clot.

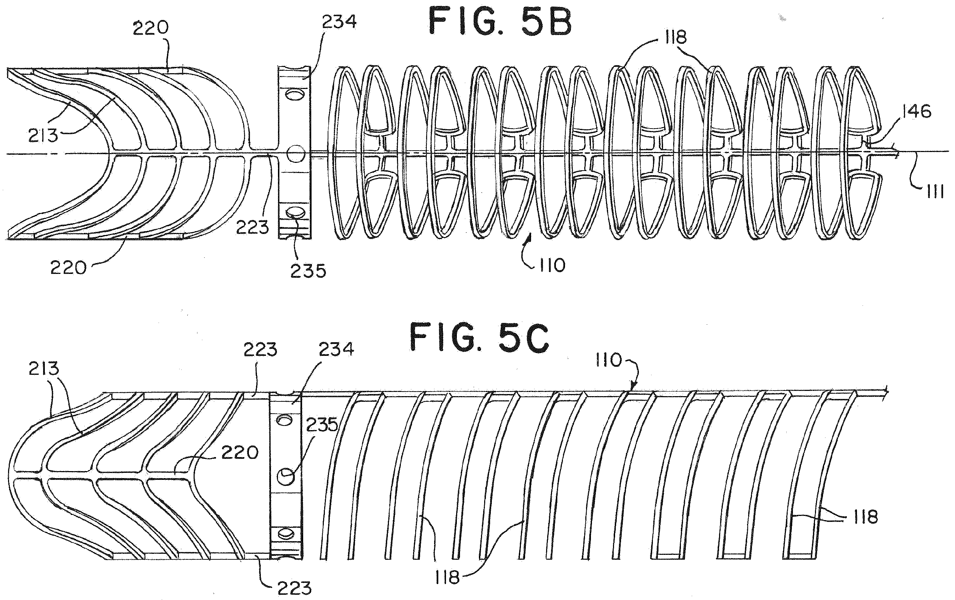

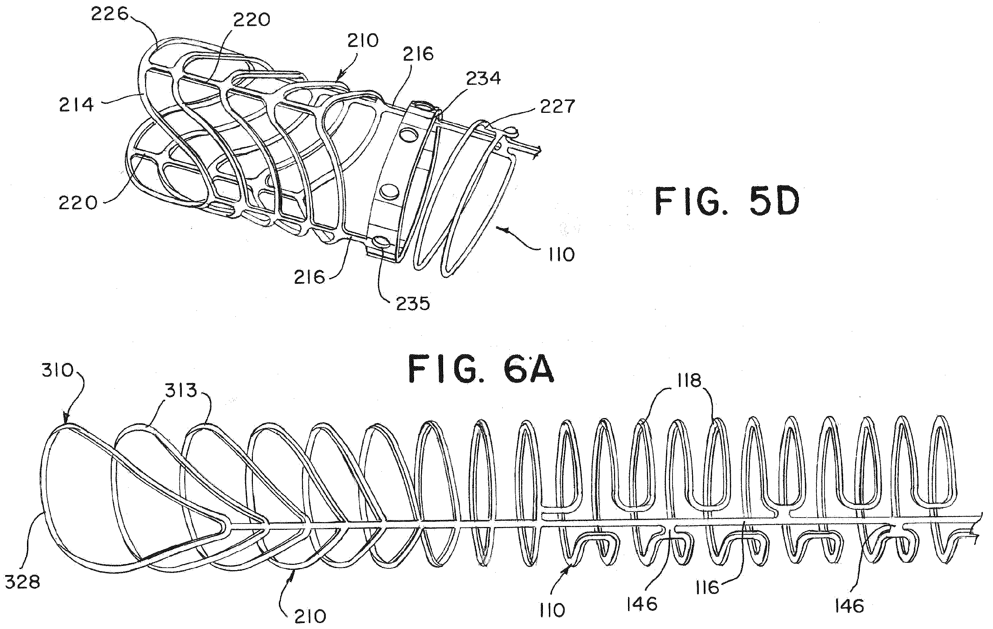

FIGs. 5A through 5D provide views of an example clot retrieval catheter 100 with an expandable support frame 210 in both collapsed delivery configurations (see FIGs. 5B through 5D) and an expanded deployed configuration (see FIG. 5A). The support frame 210 can include a connecting spine 223 that extends colinearly with respect to the axial spine 116 of the elongate body 110. The connecting spine 223 can help to position the hoop segments 213 of the support frame 210. The support frame 210 can also include a second connecting spine 223 that is diametrically opposed to the first connecting spine 223 to add additional support to the hoop segments 213.

The catheter 100 can include a support ring 234 positioned between the elongate body 110 and the support frame 210. One end of the support ring 234 can be connected to the one or more axial spines 116 of the elongate body (e.g., after the distalmost rib 227), and the other end of the support ring 234 can be connected to the one or more connecting spines 223 of the support frame 210. The support ring 234 can add structural support of the junction between the elongate body 110 and the support frame 210. The support ring 234 can also provide an indication of the location of the support frame 210 within a target vessel, for example when viewing the target site under fluoroscopy. In some examples, the support ring 234 can include radiopaque markers 235 embedded therein or otherwise attached thereto. The radiopaque markers 235 can include a denser material than the remainder of the catheter 100 so as to alert the user of the location of the support frame 210. For example, the catheter 100 can comprise a flexible material such as Nitinol, whereas the radiopaque markers 235 can include high-density materials such as gold or platinum.

In some examples, the support frame 210 can include one or more floating spines 220. The floating spine(s) 220 can be positioned at, for example, 90 degrees around the circumference of the hoop segments 213, and can provide support to the hoop segments 213 as they unfold to the expanded deployed configuration. For example, the floating spine(s) 220 can ensure the hoop segments 213 unfold into a substantially circular shape. Unlike the connecting spine(s) 223, the floating spine(s) 220 can be disconnected from the support ring 234.

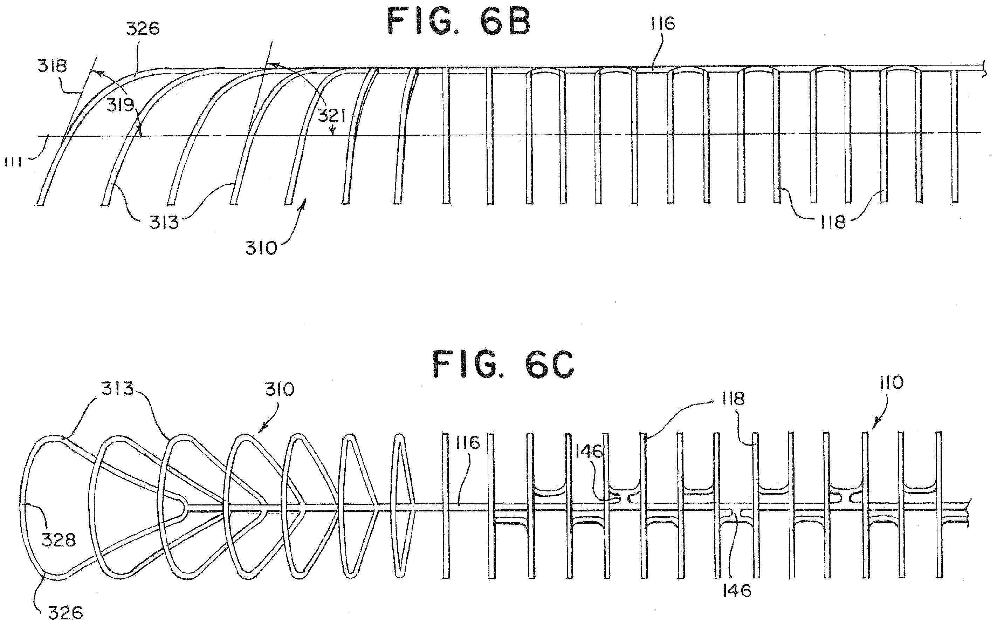

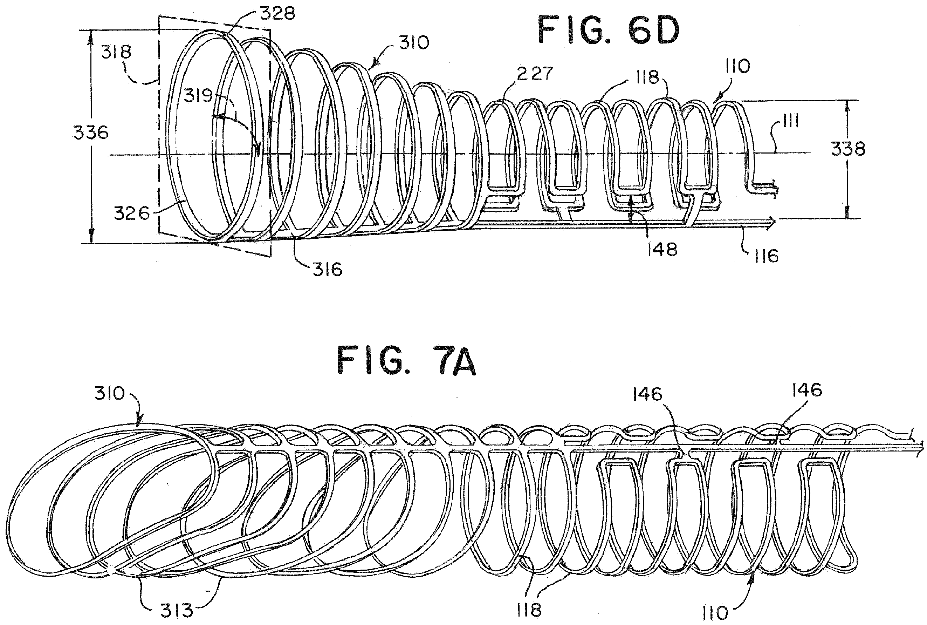

FIGs. 6A through 6D provide views of an example clot retrieval catheter 100 with an expandable support frame 310 in both collapsed delivery configurations (see FIGs. 6A through 6C) and an expanded deployed configuration (see FIG. 6D). The example catheter 100 in FIGs. 6A through 6D is similar to the example shown for FIGs. 2A through 2D, but the example in FIGs. 6A through 6D provides a variation of the support frame 310, or in particular the hoop segments (which were referred to as hoop segments 213 above). As shown, at least one hoop segment 326 of the plurality of distal hoop segments 313 can define a plane 318 passing through at least a portion of a perimeter of the at least one hoop segment 326. The plane 318 can form an acute angle 319 with respect to the longitudinal axis 111 when the distal end 114 of the elongate body 110 is in the collapsed delivery configuration. The distally unconnected peaks 328 of the distal hoop segments 313 can be spaced progressively farther apart from each other the more distal the distal hoop segments 313 are from the elongate body 110. To this end, when in the collapsed delivery configuration, a more proximal hoop segment of the distal hoop segments 313 can have a hoop folded angle 321 that is closer to 90 degrees with respect to the longitudinal axis 111 than the more distal hoop segments (e.g., hoop segment 326 at the distal end 314 of the support frame 310). This configuration can accommodate the distalmost hoop segment 326 having a first radial size 336 that is larger than the second radial size 338 of the most proximal hoop segments 313 (e.g., those proximate the elongate body 110). When the support frame 310 is unfolded into the deployed configuration (e.g., FIG. 6D), the planes 318 of the plurality of distal hoop segments 313 can all be at approximately 90 degrees with respect to the longitudinal axis 111. The axial spine 316 shown in FIGs. 6A through 6D can be similar to the axial spine 116 described herein.

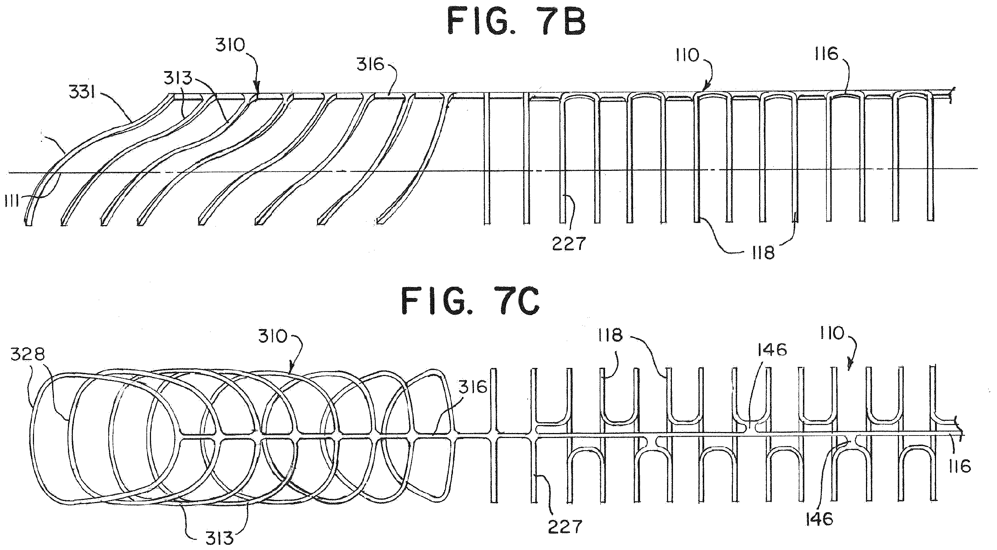

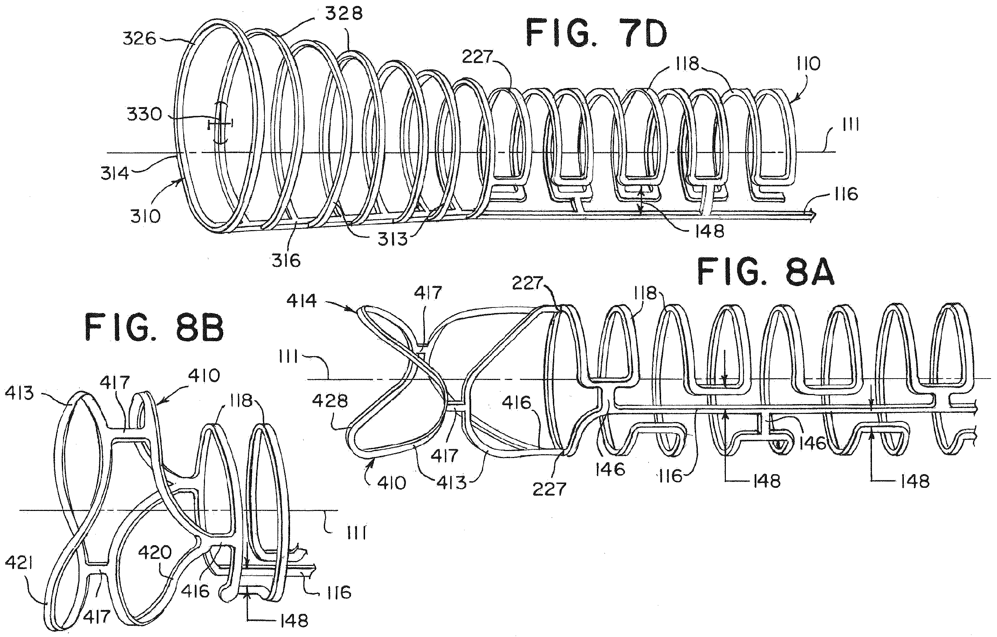

FIGs. 7A through 7D provide views of an example clot retrieval catheter 100 with an expandable support frame 310 in both collapsed delivery configurations (see FIGs. 7A through 7C) and an expanded deployed configuration (see FIG. 7D). One or more hoops of the plurality of distal hoop segments 313 can include one or more axial curves (e.g., a first axial curve 331 and a second axial curve 332) when the support frame 310 is in the collapsed delivery configuration. These axial curves 331,332 can provide a height to the circular distal hoop segments 313 such that, when they are unfolded into the expanded deployed configuration, the distal hoop segments 313 have a circular profile comprising a center 330 that is radially offset from the longitudinal axis 111 of the elongate body 110. Additionally, each hoop segment of the plurality of distal hoop segments 213 can have a non-planar cross section when the support frame 310 is in the collapsed delivery configuration.

In some examples, the elongate body 110 can include a seam 148 between certain ribs 118 of the elongate body 110 and the axial spine 116. The seam 148 can help to disconnect the ribs 118 from the axial spine 116 a certain degree to enable radial expansion of the elongate body 110 and to provide a greater degree of flexibility to the body. The seam 148 can be created by forming the ribs 118 into a serpentine shape such that only every second, third, fourth, etc. rib 118 is attached to the axial spine 116 via spine connector 146.

FIGs. 8A and 8B are perspective views of a clot retrieval catheter 100 with an expandable support frame 410, wherein the support frame 410 includes C-shaped petals 413, according to aspects of the present invention. FIG. 8A shows the example catheter 100 in a collapsed configuration, and FIG. 8B shows the example catheter 100 in an expanded deployed configuration, according to aspects of the present invention. Each pair of opposing C-shaped petals 413 can include a proximal petal 420 and a distal petal 421. The proximal petal 420 can be connected to the ribs 118 (e.g., the distalmost rib 227) via a connecting spine 416. The distal petal 421 can be connected to the proximal petal 420 via one or more linking struts 417. The proximal petal 420 and the distal petal 421 can be folded to an axially-elongated profile in the collapsed delivery configuration (see FIG. 8A). The support frame 410 can have a maximum outer diameter 224 in the expanded deployed configuration less than an inner diameter of a target vessel at a treatment site.

The C-shaped petals 413 can be clocked at 90 degrees to each other for improved flexibility and enhanced ability to recover and maintain the shape and inner diameter of the support frame 410 when displaced laterally in a vessel. Additionally, a clocking offset between the connecting spine 416 of the support frame 410 and the axial spine 116 of the elongate body 110 can have a hinge effect allowing the structure to easily deflect away from vessel walls. As shown, the opposing C-shaped petals 413 can include distally unconnected peaks 428 at a distal end 414 of the support frame 410.

FIG. 9 is a side perspective view of a clot retrieval catheter 100 with an expandable support frame 410 in a collapsed delivery configuration, wherein the support frame 410 includes a circumferential undulation 418, according to aspects of the present invention. The catheter 100 shown in FIG. 9 is similar to the embodiment shown in FIGs. 8A and 8B but with variations to the support frame 410. The one or more circumferential undulations 418 on the support frame 410 can flare with a large bend radius for atraumatic contact with vessel walls. The support frame 410 can further include one or more intermediate hoops 429 extending from the C-shaped petals 413. The intermediate hoops 429 can provide additional scaffolding for a membrane (e.g., jacket 180 shown in FIG. 10) that is attached to the catheter 100 to direct fluid and/or the clot into the elongate body 110. Extending from the proximal ring member 434 can be a base ring support hoop 435, which can be separate from the proximal petal 420 and provide additional scaffolding for a membrane (e.g., jacket 180 shown in FIG. 10).

FIG. 10 is a perspective view of a clot retrieval catheter 100 with an expandable support frame 410 and a jacket 180 in an expanded deployed configuration. The jacket 180 can be a polymer jacket extrusion which can be reflowed or laminated in place. The applied heat can allow the outer polymer to fill the interstitial sites between the ribs 118 of the elongate body 110. Suitable jacket 180 materials can include elastic polyurethanes such as Chronoprene, which can have a shore hardness of 40A or lower, silicone elastomers, or similar materials.

The jackets 180 can also be applied in a combination of other ways. Depending on their axial location on the elongate body 110 and/or support frames (e.g., support frame 210,310,410) the jacket 180 can be dip coated, sprayed, electro spun, and/or plasma deposited onto the support frame. In other examples, the jacket 180 can be a straight extrusion or extruded and post-formed onto the expanding tip and catheter body.

In order to allow for smooth delivery of the clot retrieval catheter 100 through an outer catheter, the outer surface of the jacket 180 can be coated with a low-friction or lubricious material, such as PTFE or commercially available lubricious coating.

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. As a result, not all possible combinations have been listed, and such variants are often apparent to those of skill in the art and are intended to be within the scope of the claims which follow. It is intended that each term contemplates its broadest meaning as understood by those skilled in the art and includes all technical equivalents that operate in a similar manner to accomplish a similar purpose without departing from the scope of the invention. It is also to be understood that the mention of one or more steps of a method does not preclude the presence of additional method steps or intervening method steps between those steps expressly identified. Similarly, some steps of a method can be performed in a different order than those described herein without departing from the scope of the disclosed technology.

The invention is defined by the following claims.

## Claims

1. A catheter (100) comprising:
an elongate body (110) comprising a longitudinal axis (111), an inner diameter (115), and a distal end; and
a support frame (210) connected at the distal end of the elongate body comprising a collapsed delivery configuration, an expanded deployed configuration, and a framework of struts comprising a plurality of distal hoop segments (213),
at least a portion of the distal hoop segments folding distally in the collapsed delivery configuration such that a collapsed inner diameter (215) of the support frame is approximately equal to the inner diameter (115) of the elongate body,
wherein a first more distal hoop segment of the plurality of distal hoop segments comprising a first diameter when the support frame is in the expanded deployed configuration and a second more proximal hoop segment of the plurality of distal hoop segments forming a second diameter when the support frame is in the expanded deployed configuration, the first diameter being greater than the second diameter, and
the plurality of distal hoop segments comprising distally unconnected peaks (228) which move distally when the support frame is folded to the collapsed delivery configuration.

2. The catheter of claim 1, the struts of the distal hoop segments comprising a curvilinear profile in the collapsed delivery configuration.

3. The catheter of claim 1, the support frame further comprising a longitudinal length sized to be less than three times the inner diameter of the elongate body.

4. The catheter of claim 1, further comprising one or more connecting spines connecting the distal hoop segments of the support frame with the elongate body, and, optionally, wherein the elongate body comprising a plurality of ribs (118) and one or more axial spines (116), and at least one of the one or more axial spines being aligned with a connecting spine of the support frame.

5. The catheter of claim **1,** the plurality of distal hoop segments folding so the support frame comprises a collapsed inner diameter in the collapsed delivery configuration approximately equal to the inner diameter of the elongate body.

6. The catheter of claim **1,** the distally unconnected peaks moving proximally when the support frame is in the expanded deployed configuration .

7. The catheter of claim **1,** a first more distal hoop segment of the plurality of distal hoop segments forming a first folded angle with respect to the longitudinal axis when the support frame is in the collapsed delivery configuration,
a second more proximal hoop segment of the plurality of distal hoop segments forming a second folded angle with respect to the longitudinal axis when the support frame is in the collapsed delivery configuration, and
the first folded angle being less than the second folded angle.

8. The catheter of claim **1,** the plurality of distal hoop segments forming a series of rings concentric with the longitudinal axis when the support frame is in the expanded deployed configuration.

9. The catheter of claim **1,** at least a portion of each of the plurality of distal hoop segments forming an acute angle with respect to the longitudinal axis when the support frame is in the collapsed delivery configuration.

10. The catheter of claim **1,** each hoop segment of the plurality of distal hoop segments comprising a non-planar cross section when the support frame is in the collapsed delivery configuration.

11. The catheter of claim 1, one or more hoops of the plurality of distal hoop segments further comprising one or more axial curves (331, 332) when the support frame is in the collapsed delivery configuration.

## Patentansprüche

1. Katheter (100), umfassend:
einen länglichen Körper (110), umfassend eine Längsachse (111), einen Innendurchmesser (115) und ein distales Ende; und
einen Stützrahmen (210), der an dem distalen Ende des länglichen Körpers verbunden ist, umfassend eine kollabierte Zuführkonfiguration, eine expandierte entfaltete Konfiguration und ein Gerüst aus Streben, umfassend eine Vielzahl von distalen Ringsegmenten (213),
wobei sich mindestens ein Abschnitt der distalen Ringsegmente in der kollabierten Zuführkonfiguration derart distal faltet, dass ein kollabierter Innendurchmesser (215) des Stützrahmens etwa gleich dem Innendurchmesser (115) des länglichen Körpers ist,
ein erstes mehr distales Ringsegment der Vielzahl von distalen Ringsegmenten umfassend einen ersten Durchmesser, wenn sich der Stützrahmen in der expandierten entfalteten Konfiguration befindet, und ein zweites mehr proximales Ringsegment der Vielzahl von distalen Ringsegmenten einen zweiten Durchmesser ausbildet, wenn sich der Stützrahmen in der expandierten entfalteten Konfiguration befindet, wobei der erste Durchmesser größer als der zweite Durchmesser ist, und
die Vielzahl von distalen Ringsegmenten umfassend distal unverbundene Spitzen (228), die sich distal bewegen, wenn der Stützrahmen in die kollabierte Zuführkonfiguration gefaltet wird.

2. Katheter nach Anspruch 1, die Streben der distalen Ringsegmente umfassend ein krummliniges Profil in der kollabierten Zuführkonfiguration.

3. Katheter nach Anspruch 1, der Stützrahmen ferner umfassend eine Längslänge, die dimensioniert ist, um kleiner als ein Dreifaches des Innendurchmessers des länglichen Körpers zu sein.

4. Katheter nach Anspruch 1, ferner umfassend eine oder mehrere Verbindungsgrate, die die distalen Ringsegmente des Stützrahmens mit dem länglichen Körper verbinden, und, optional, der längliche Körper umfassend eine Vielzahl von Rippen (118) und eine oder mehrere axiale Grate (116), und wobei mindestens einer des einen oder der mehreren axialen Grate mit einem Verbindungsgrat des Stützrahmens ausgerichtet ist.

5. Katheter nach Anspruch 1, wobei sich die Vielzahl von distalen Ringsegmenten faltet, sodass der Stützrahmen in der kollabierten Zuführkonfiguration einen kollabierten Innendurchmesser umfasst, der etwa gleich dem Innendurchmesser des länglichen Körpers ist.

6. Katheter nach Anspruch 1, wobei sich die distal unverbundenen Spitzen proximal bewegen, wenn sich der Stützrahmen in der expandierten entfalteten Konfiguration befindet.

7. Katheter nach Anspruch 1, wobei ein erstes mehr distales Ringsegment der Vielzahl von distalen Ringsegmenten einen ersten gefalteten Winkel in Bezug auf die Längsachse ausbildet, wenn sich der Stützrahmen in der kollabierten Zuführkonfiguration befindet,
ein zweites mehr proximales Ringsegment der Vielzahl von distalen Ringsegmenten einen zweiten gefalteten Winkel in Bezug auf die Längsachse ausbildet, wenn sich der Stützrahmen in der kollabierten Zuführkonfiguration befindet, und
wobei der erste gefaltete Winkel kleiner als der zweite gefaltete Winkel ist.

8. Katheter nach Anspruch 1, wobei die Vielzahl von distalen Ringsegmenten eine Reihe von Ringen ausbildet, die zu der Längsachse konzentrisch sind, wenn sich der Stützrahmen in der expandierten entfalteten Konfiguration befindet.

9. Katheter nach Anspruch 1, wobei mindestens ein Abschnitt jedes der Vielzahl von distalen Ringsegmenten einen spitzen Winkel in Bezug auf die Längsachse ausbildet, wenn sich der Stützrahmen in der kollabierten Zuführkonfiguration befindet.

10. Katheter nach Anspruch 1, jedes Ringsegment der Vielzahl von distalen Ringsegmenten umfassend einen nicht planaren Querschnitt, wenn sich der Stützrahmen in der kollabierten Zuführkonfiguration befindet.

11. Katheter nach Anspruch 1, ein oder mehrere Ringe der Vielzahl von distalen Ringsegmenten ferner umfassend eine oder mehrere axiale Krümmungen (331, 332), wenn sich der Stützrahmen in der kollabierten Zuführkonfiguration befindet.

## Revendications

1. Cathéter (100) comprenant :
un corps allongé (110) comprenant un axe longitudinal (111), un diamètre interne (115), et une extrémité distale ; et
une armature de support (210) reliée à l'extrémité distale du corps allongé comprenant une configuration de pose repliée, une configuration déployée expansée, et une structure d'entretoises comprenant une pluralité de segments d'arceau distaux (213),
au moins une partie des segments d'arceau distaux se repliant distalement dans la configuration de pose repliée de telle sorte qu'un diamètre interne replié (215) de l'armature de support soit approximativement égal au diamètre interne (115) du corps allongé,
dans lequel un premier segment d'arceau plus distal de la pluralité de segments d'arceau distaux comprend un premier diamètre lorsque l'armature de support est dans la configuration déployée expansée et un second segment d'arceau plus proximal de la pluralité de segments d'arceau distaux forme un second diamètre lorsque l'armature de support est dans la configuration déployée expansée, le premier diamètre étant supérieur au second diamètre, et
la pluralité de segments d'arceau distaux comprenant des sommets non reliés distalement (228) qui se déplacent distalement lorsque l'armature de support est repliée vers la configuration de pose repliée.

2. Cathéter selon la revendication 1, les entretoises des segments d'arceau distaux comprenant un profil curviligne dans la configuration de pose repliée.

3. Cathéter selon la revendication 1, l'armature de support comprenant en outre une longueur longitudinale dimensionnée pour être inférieure à trois fois un diamètre interne du corps allongé.

4. Cathéter selon la revendication 1, comprenant en outre une ou plusieurs arêtes de liaison reliant les segments d'arceau distaux de l'armature de support au corps allongé, et, éventuellement, dans lequel le corps allongé comprend une pluralité de nervures (118) et une ou plusieurs arêtes axiales (116), et au moins l'une de la ou des arêtes axiales étant alignée avec une arête de liaison de l'armature de support.

5. Cathéter selon la revendication 1, la pluralité de segments d'arceau distaux se repliant de sorte que l'armature de support comprenne un diamètre interne replié dans la configuration de pose repliée approximativement égal au diamètre interne du corps allongé.

6. Cathéter selon la revendication 1, les sommets non reliés distalement se déplaçant proximalement lorsque l'armature de support est dans la configuration déployée expansée.

7. Cathéter selon la revendication 1, un premier segment d'arceau plus distal de la pluralité de segments d'arceau distaux formant un premier angle de pliage par rapport à l'axe longitudinal lorsque l'armature de support est dans la configuration de pose repliée,
un second segment d'arceau plus proximal de la pluralité de segments d'arceau distaux formant un second angle de pliage par rapport à l'axe longitudinal lorsque l'armature de support est dans la configuration de pose repliée, et
le premier angle de pliage étant inférieur au second angle de pliage.

8. Cathéter selon la revendication 1, la pluralité de segments d'arceau distaux formant une série d'anneaux concentriques à l'axe longitudinal lorsque la structure de support est dans la configuration déployée expansée.

9. Cathéter selon la revendication 1, au moins une partie de chacun de la pluralité de segments d'arceau distaux formant un angle aigu par rapport à l'axe longitudinal lorsque la structure de support est dans la configuration de pose repliée.

10. Cathéter selon la revendication 1, chaque segment d'arceau de la pluralité de segments d'arceau distaux comprenant une section transversale non plane lorsque l'armature de support est dans la configuration de pose repliée.

11. Cathéter selon la revendication 1, un ou plusieurs arceaux de la pluralité de segments d'arceau distaux comprenant en outre une ou plusieurs courbes axiales (331, 332) lorsque l'armature de support est dans la configuration de pose repliée.
